# EUROPEAN PATENT SPECIFICATION

(11) **EP 4 424 260 B1**
(45) Date of publication and mention of the grant of the patent: **17.09.2025**
(21) Application number: 24152394.3
(22) Date of filing: 17.01.2024
(51) Int. Cl.: A61B 18/14, A61B 34/00, A61B 5/00

(54) **HEURISITIC SELECTION OF DENERVATION THERAPY**
HEURISITISCHE AUSWAHL EINER DENERVATIONSTHERAPIE
SÉLECTION HEURISTIQUE D'UNE THERAPIE DE DÉNERVATION

(30) Priority: 28.02.2023 US 202363487390 P
(43) Date of publication of application: 04.09.2024
(73) Proprietor: Medtronic Ireland Manufacturing Unlimited Company, Dublin 2, D02 T380 (IE)
(72) Inventor: Coates, Paul J., Santa Rosa, 95403 (US); Tunev, Stefan S., Santa Rosa, 95403 (US)
(74) Representative: Zimmermann & Partner Patentanwälte mbB

(56) References cited:
- WO-A1-2019/210064
- US-A1- 2019 223 955
- US-A1- 2021 383 908

## Description

### TECHNICAL FIELD

This disclosure generally relates to selection of denervation therapy.

### BACKGROUND

Percutaneous renal denervation is a procedure that can be used for treating hypertension. During a renal denervation procedure, a device delivers stimuli or energy, such as radiofrequency, ultrasound, cooling or other energy, to a treatment site to reduce activity of nerves surrounding a blood vessel. The stimuli or energy delivered to the treatment site may provide various therapeutic effects through alteration of sympathetic nerve activity. Percutaneous denervation of the afferent renal nerves, efferent renal nerves, or both may result in blood pressure (BP) lowering in some patients with uncontrolled hypertension in both the presence and absence of concomitant anti-hypertensive drug therapy.

WO 2019/210064 A1 describes devices, systems, and techniques for predicting renal denervation efficacy in reducing hypertension in a patient based on pulse information from one or more wrists of the patient. US 2021/383908 A1 relates to a device for determining a renal response index which provides valuable information regarding a possible renal denervation process. US 2019/223955 A1 describes devices, systems, and techniques for determining one or more parameters of denervation therapy, which may also be referred to as neuromodulation therapy.

### SUMMARY

Denervation therapy may provide a therapeutic benefit to certain patients. For example, renal denervation may mitigate symptoms associated with renal sympathetic nerve overactivity. Various aspects of the techniques described in this disclosure relate to a computing device determining a targeted ablation strategy based on a complexity score of the renal arterial anatomy. As such, the various aspects of the techniques may reduce a number of ablations being performed while achieving a desired result for the patient, which in turn improves health care efficiency, while also reducing patient discomfort in undergoing an RDN procedure to deliver denervation therapy. Renal arterial anatomy information for a respective kidney may be received via an aortogram, CT scan, or other imaging devices.

Accordingly, the present disclosure describes a non-invasive predictor to determine, before performing an RDN procedure to deliver denervation therapy, which type of renal denervation therapy may achieve a desired ablation result in a patient, while reducing unnecessary denervation or unnecessarily complicated denervation. This determination may help identify which patients may benefit from a simpler ablation procedure, such as applying one or more ablations only to a trunk of a main renal artery and which patients benefit from a more complex ablation procedure, such as applying ablations to a trunk of the main renal artery and one or more primary and/or secondary branches of the main renal artery. For instance, a computing device may determine the type of renal denervation therapy before an invasive denervation procedure is performed, which may improve health care efficiency and patient care by reducing performance of ineffective procedures (e.g., performing simpler ablation procedure on a patient who would benefit from a more complex ablation procedure) and/or reducing patient discomfort from undergoing an unnecessary complex ablation procedure (e.g., performing a complex ablation procedure on a patient that only requires a simpler ablation procedure).

In one example, this disclosure is directed to a system comprising: a memory; and processing circuitry coupled to the memory, the processing circuitry configured to: receive renal arterial anatomy information of a patient; determine a renal anatomical complexity score based on the renal arterial anatomy information; determine a type of renal denervation therapy to apply to the patient based on the renal anatomical complexity score; and output an indication of the determined type of renal denervation therapy to apply to the patient.

In another example, this disclosure is directed to a method comprising: receiving renal arterial anatomy information of a patient; determining a renal anatomical complexity score based on the renal arterial anatomy information; determining a type of renal denervation therapy to apply to the patient based on the renal anatomical complexity score; and outputting an indication of the determined type of renal denervation therapy to apply to the patient.

Further details of one or more examples of this disclosure are set forth in the accompanying drawings and in the description below. Other features, objects, and advantages will be apparent from the description and drawings, and from the claims.

The above summary is not intended to describe each illustrated example or every implementation of the present disclosure.

### BRIEF DESCRIPTION OF DRAWINGS

FIG. 1 is a conceptual diagram illustrating an example system for determining a type of denervation therapy to apply to a patient, in accordance with some examples of the current disclosure.
FIG. 2 is a block diagram illustrating an example configuration of a computing device and an imaging device, in accordance with some examples of the current disclosure.
FIGS. 3A-3B are conceptual diagrams of renal arterial anatomy information accordance with some examples of the current disclosure.
FIG. 4 is a conceptual diagram illustrating an example neural network configured to determine a type of renal denervation therapy to apply to a patient.
FIG. 5 is a flow diagram illustrating an example technique for operating a system to determine a type of renal denervation therapy to apply to a patient.

### DETAILED DESCRIPTION

Denervation therapy, such as RDN therapy, may be used to render a nerve inert, inactive, or otherwise completely or partially reduced in function, such as by ablation or lesioning of the nerve. Following denervation, there may be a reduction or even prevention of neural signal transmission along the target nerve. Denervating an overactive nerve may provide a therapeutic benefit to a patient. For example, renal denervation may mitigate symptoms associated with renal sympathetic nerve overactivity, such as hypertension. Denervation therapy may include delivering electrical and/or thermal energy to a target nerve, and/or delivering a chemical agent to a target nerve. In the case of renal denervation therapy, the denervation energy or chemical agents can be delivered, for example, via a therapy delivery device (e.g., a catheter) disposed in a blood vessel (e.g., the renal artery) proximate to the renal nerve.

The renal sympathetic nervous system has been identified as a potential major contributor to the complex pathophysiology of hypertension, or elevated systemic blood pressure (BP). Therefore, renal denervation may reduce renal sympathetic nerve overactivity and cause a reduction in systemic BP as a treatment for hypertension. In some patients, renal denervation may reduce office systolic BP in a range of approximately 5 millimeters of mercury (mmHg) to 30 mmHg. However, renal denervation may not reduce systolic BP for other patients. RDN therapy may be used as treatment for other ailments associated with changes in sympathetic nervous system activity as well, such as arrhythmias and heart failure.

One approach for RDN therapy is to ablate as much of the renal artery 'tree' as possible within the allowable range of vessel diameter and location of ablation site relative to the kidney. Although this approach has been shown to be safe and efficacious, some of these ablations may be unnecessary for some patients. Performing unnecessary ablations may cause unnecessarily longer RDN procedures, unnecessary higher costs of RDN therapy, unnecessary patient inconvenience and discomfort, and/or unnecessary potential side effects.

In some examples of the present technique, processing circuitry (e.g., of a computing device) may determine a targeted ablation strategy based on the complexity of the renal arterial anatomy. Processing circuitry may select a type of RDN ablation treatment based on complexity scores of the renal arterial anatomy of one or more kidneys. The complexity score may use any rating system, such as, but not limited, to a numerical rating system.

As described in more detail, the "complexity" of the renal arterial anatomy may include information such as whether a length of a main renal artery is less than a lower threshold, greater than upper threshold, or between the lower and upper threshold. In some examples, a main renal artery may be a blood vessel that feeds the largest volume of blood from the aorta to a respective kidney.

In some examples, there may be more than one blood vessel that feeds blood from the aorta to the respective kidney. For example, if two or more blood vessels each feed a similar amount of volume of blood to the respective kidney, and those respective two or more blood vessels each respectively feed the largest volume of blood to the respective kidney, and each respectively feed a volume of blood from the aorta to the respective kidney above a main renal artery volume threshold, those two or more blood vessels that respectively feed the largest volume of blood to the respective kidney may each be identified as a respective main renal artery.

In some examples, when there are additional blood vessels that feed blood from the aorta to the kidney, but feed a volume of blood lower than a volume of blood fed by the main renal artery, and the volume of blood being fed by the additional blood vessels being below a main renal artery volume threshold, those particular additional blood vessels may be identified as accessory renal arteries. In some examples, accessory arteries may originate from the aorta or vessels distal of the aorta such as the iliac artery. In some examples, accessory arteries may originate superior or inferior to the origin of the main renal artery.

In some examples, a length of a main renal artery may refer to a portion of a renal blood vessel originating from the aorta to a first or primary bifurcation point. In some examples, a portion of the main renal artery from the aorta or main renal artery ostium (typically located at the aorta) to a first or primary bifurcation point may be referred to as a "trunk" of that particular main renal artery, while the portions of the blood vessel past the first or primary bifurcation point may be referred to as "branches" of the main renal artery.

As another example, the complexity may be whether the number of renal arteries is less than or greater than a renal artery count threshold. As another example, the complexity may be based on a shape and/ or configuration of a main renal artery. In general, the complexity of the renal arterial anatomy refers to information indicative of classification of the structure of the renal arterial anatomy, and the above examples of the complexity of the renal arterial anatomy are provided as non-limiting examples.

The present disclosure describes various aspects of techniques related to determining a patient unique targeted ablation strategy based on the complexity of the renal arterial anatomy of the patient who will undergo the RDN procedure. While the above examples discuss renal denervation responsiveness in reducing hypertension, the techniques described herein may similarly be used to determine renal denervation responsiveness for treatment of other ailments associated with changes in sympathetic nervous system activity, such as arrhythmias and heart failure.

While the above examples discuss determining a patient unique targeted ablation strategy based on the complexity of the renal arterial anatomy of the patient who will undergo the RDN procedure, the techniques described herein may similarly be used to determine a denervation strategy based on arterial anatomy at other locations, such as at or around a pulmonary artery, hepatic artery, celiac artery, mesenteric artery, arteries that feed a digestive system, arteries that feed a reproductive system, and/or other arteries. In some examples, the techniques described herein may similarly be used, based on arterial anatomy at multiple locations, to determine a denervation strategy at multiple locations, such as two or more of a main renal artery, a pulmonary artery, hepatic artery, celiac artery, mesenteric artery, arteries that feed a digestive system, arteries that feed a reproductive system, and/or other arteries.

For example, a computing device or system may receive renal arterial anatomy information for a respective kidney of a patient. In some examples, renal arterial anatomy may be received via an aortogram, computerized tomography (CT) scan, or other devices. The computing device or system may determine an anatomical complexity score based on the received arterial anatomy information. The computing device or system may determine a type of renal denervation therapy to apply to the patient based on the anatomical complexity score.

Determining a patient unique targeted ablation strategy, before performing a denervation procedure, in accordance with various techniques of this disclosure, may help prevent clinicians from performing unnecessary ablations on patients that are not necessary to achieve a desired clinical result. This may help reduce unnecessary health care costs by reducing RDN procedure time in some patients while also reducing unnecessary discomfort on patients by reducing unnecessary ablations.

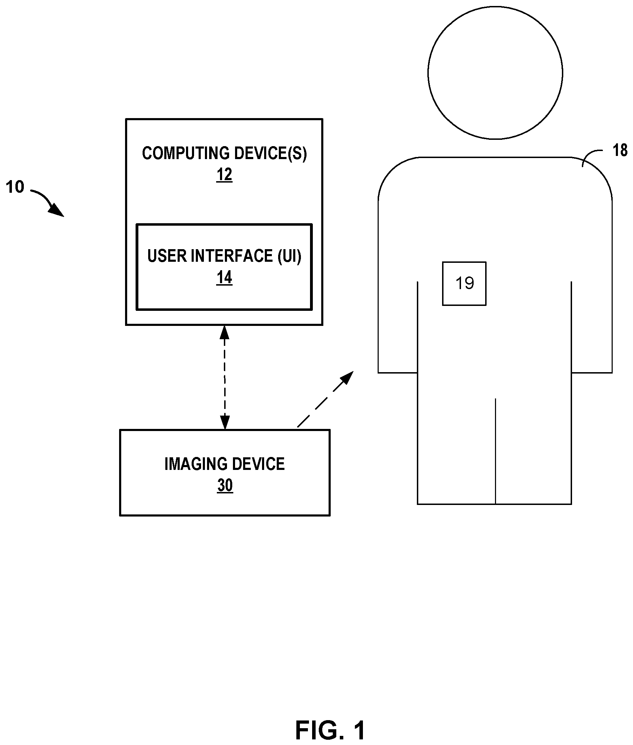

FIG. 1 is a conceptual diagram illustrating an example system 10 for determining a type of denervation therapy to apply to a patient, in accordance with some examples of the current disclosure. As shown in FIG. 1, system 10 includes a computing device 12. Computing device 12 may be a computing device used in a home, ambulatory, clinic, or hospital setting. Computing device 12 may include, for example, a clinician programmer, a desktop computer, a laptop computer, a workstation, a server, a mainframe, a cloud computing system, a smartphone, combinations thereof, or the like. Computing device 12 may be configured to receive, via a user interface device 14 ("UI 14"), input from a user, such as a clinician, output information to a user, or both. In some examples, UI 14 may include a display (e.g., a liquid crystal display (LCD) or light emitting diode (LED) display), such as a touch-sensitive display; one or more buttons; one or more keys (e.g., a keyboard); a mouse; one or more dials; one or more switches; a speaker; one or more lights; combinations thereof; or the like. Imaging device 30 may be an imaging modality that may collect imaging data of anatomy of patient 18, such as arterial anatomy. In some examples, imaging device 30 may collect imaging data of renal arterial anatomy 19 of patient 18. Computing device 12 may receive the collected imaging data and determine a type of RDN therapy to apply based, at least in part, on the collected imaging data.

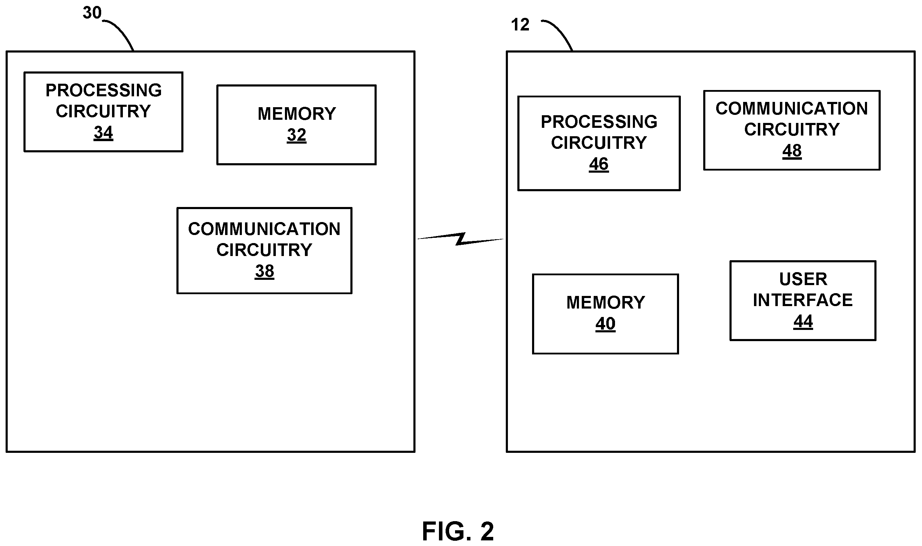

FIG. 2 is a block diagram illustrating an example configuration of computing device 12 of FIG. 1 and an imaging device 30. As shown in FIG. 2, imaging device 30 may include processing circuitry 34, memory 32, and communication circuitry 38. As shown in FIG. 2, computing device 12 may include processing circuitry 46, communication circuitry 48, memory 40, and UI 14. Memory 32 or 40 may include any volatile or non-volatile media, such as a random access memory (RAM), read only memory (ROM), non-volatile RAM (NVRAM), electrically erasable programmable ROM (EEPROM), flash memory, or the like. Memory 32 or 40 may store computer-readable instructions that, when executed by processing circuitry 34 or 46, respectively, cause imaging device 30 or computing device 12, respectively, to perform various functions described herein. Processing circuitry 34 or 46 may include any combination of one or more processors including one or more microprocessors, digital signal processors (DSPs), application specific integrated circuits (ASICs), field programmable gate arrays (FPGAs), or other equivalent integrated or discrete logic circuitry. Accordingly, processing circuitry 34 or 46 may include any suitable structure, whether in hardware, software, firmware, or any combination thereof, to perform the functions ascribed herein to processing circuitry 34 or 46. Computing device 12 and imaging device 30 may be configured to receive data (e.g., via communication circuitry 48 or 38) from another computing device. In some examples, computing device 12 and imaging device 30 may be separate devices. In some examples, computing device 12 and imaging device 30 may be located on the same device.

Computing device 12 may receive arterial anatomy information of patient 18. Examples of arterial anatomy information of patient 18 include images and/or imaging data of arterial anatomy. Other examples of arterial anatomy information of patient 18 may include one or more of textual information indicative of relative locations of structures within the arterial anatomy, length of main renal artery, diameter of main renal artery, main renal artery wall thickness, main renal artery wall stiffness, bifurcation location relative to aorta to hilum distance, relative height of main renal artery take-off (for example, using vertebrae as measure of height), location where main renal artery enters kidney (number of access points away from renal hilum), ratios/differences in main renal artery wall thickness at different locations, and/or ratios/differences of main renal artery wall stiffness at different locations. Other examples of arterial anatomy information may include accessory artery count, accessory artery take-off relative to main renal artery (inferior, superior, ostial separation distance).

In some examples, arterial anatomy information of patient 18 may include or further include one or more of length of main pulmonary, hepatic, or other artery, diameter of main pulmonary, hepatic, or other artery, main pulmonary, hepatic, or other artery wall thickness, main pulmonary, hepatic, or other artery wall stiffness, bifurcation location relative to pulmonary, hepatic, or other artery distance, relative height of main pulmonary, hepatic, or other artery take-off (for example, using vertebrae as measure of height), location where main pulmonary, hepatic, or other artery enters organ, ratios/differences in main pulmonary, hepatic, or other artery wall thickness at different locations, and/or ratios/differences of main pulmonary, hepatic, or other artery wall stiffness at different locations. Other examples of arterial anatomy information may include accessory artery count, accessory artery take-off relative to main pulmonary, hepatic, or other artery (inferior, superior, ostial separation distance).

In some examples, imaging device 30 may collect renal arterial anatomy information for a respective kidney via an aortogram, CT scan, or other imaging modalities. Computing device 12 may receive the collected renal arterial anatomy information from imaging device 30. For instance, imaging device 30 may capture images or imaging data of the renal arterial anatomy, and transmit such images to computing device 12. In some examples, contrast may be released in an aorta of patient 18, which then flows downstream and feeds into the renal arteries, to obtain renal arterial anatomy information for a respective kidney via an aortogram or CT scan, which are examples of the images that imaging device 30 may capture and/or generate.

In some examples, computing device 12 may receive renal arterial anatomy information via another computing device, such as a server, storing renal arterial anatomy information of patient 18. For example, computing device 12 may receive CT image data of renal arterial anatomy from a health records system or server. In some examples, computing device 12 may store renal arterial anatomy information of patient 18, e.g., from a previously obtained image. In some examples, imaging device 30 may identify and collect renal arterial anatomy information of patient 18.

In some examples, imaging device 30 may store renal arterial anatomy information in memory 32. Imaging device 30 may be communicatively coupled to computing device 12 and may transmit collected renal arterial anatomy information to computing device 12 via communication circuitry 38, either directly or via other computing devices. In some examples, the renal arterial anatomy information of patient 18 may be received by computing device 12 before an RDN catheter is introduced into patient 18 to perform a denervation procedure.

Determining a patient unique RDN therapy, before performing RDN therapy, may help prevent clinicians from performing unnecessary ablations on patients that are not necessary to achieve a desired clinical result. This may help reduce unnecessary health care costs by reducing RDN procedure time in some patients while also reducing unnecessary discomfort on patients by reducing unnecessary ablations.

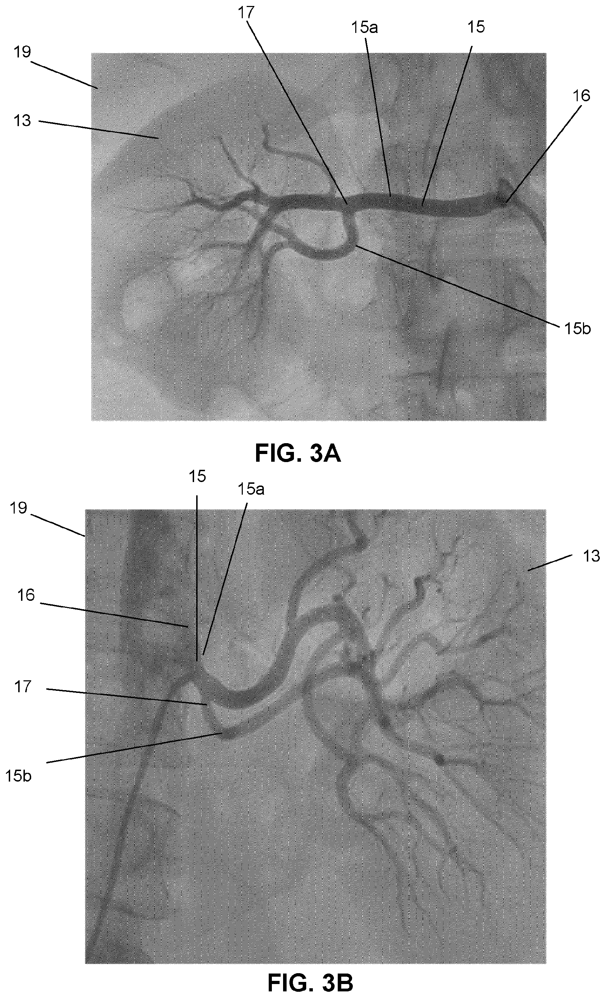

The arterial anatomy information may include one or more of a length of a main renal artery or a number of main renal arteries. FIGS. 3A-3B show examples of renal arterial anatomy information 19. In some examples, a length of main renal artery 15 may be the length of the artery from the main renal artery ostium 16 (e.g., typically located at the aorta) to a first or primary bifurcation point 17. In some examples, there may be one main renal artery 15 for a respective kidney 13. In some examples, there may be multiple main renal arteries for a respective kidney 13. In some examples, renal arterial anatomy information may further include a distance from the main renal artery ostium 16 to a kidney hilum. In some examples, a portion of the main renal artery 15 from the aorta or main renal artery ostium 16 to a first or primary bifurcation point 17 may be referred to as a trunk 15a of main renal artery 15, while the portions of the blood vessel past the first or primary bifurcation point 17 may be referred to as a branch 15b of main renal artery 15.

Processing circuitry 46 may perform image processing of the collected imaging data to extract various features of the renal arterial anatomy information from the imaging data, such as a length of the main renal artery and/or a number of renal arteries present for the respective kidney.

In some examples, to perform image processing of the collected imaging data to extract various features of the renal arterial anatomy information from the imaging data, processing circuitry 46 may determine whether image quality of the collected imaging data is sufficient to complete a segmentation process. Processing circuitry 46 may perform a segmentation process on the collected imaging data, such as identifying key structures and/or locations of the collected imaging data, such as arteries, bifurcations, and/or kidney. Processing circuitry 46 may assign numerical values to the segmented image, such as main renal artery length, main renal artery diameter, main renal artery location, and/or a count of main renal arteries.

Processing circuitry 46 may determine a renal anatomical complexity score based on one or more of a length of the main renal artery 15 or a number of main renal arteries present for the respective kidney 13. For example, processing circuitry 46 may determine main renal artery length anatomical complexity score based on a length of the main renal artery and/or determine a main renal artery count anatomical complexity score based on the number of main renal arteries. Processing circuitry 46 may then determine a renal anatomical complexity score based on the determined main renal artery length anatomical complexity score and/or main renal artery count anatomical complexity score.

The main renal artery length from the main renal artery ostium 16 to the first or primary bifurcation point 17 may be inversely related to the main renal artery length anatomical complexity score. For example, if the main renal artery length is greater than a particular main renal artery length threshold, processing circuitry 46 may determine the main renal artery length anatomical complexity score is low. If the main renal artery length is less than the main renal artery length threshold, processing circuitry 46 may determine the main renal artery length anatomical complexity score is high.

For example, if the length of the main renal artery 15 is determined to be greater than or equal to an upper main renal artery length threshold, such as 25mm, processing circuitry 46 may determine the main renal artery length anatomical complexity score is given its lowest value, such as "1". If the length of the main renal artery 15 is determined to be less than or equal to a lower main renal artery length threshold, such as 15mm, processing circuitry 46 may determine the main renal artery length anatomical complexity score is given its highest value, such as "3". If the length of the main renal artery 15 is determined to be greater than the lower main renal artery length threshold and less than the upper main renal artery length threshold, processing circuitry 46 may determine the main renal artery length anatomical complexity score is given a value between its highest value and lowest value, such as "2". The upper main renal artery length threshold and lower main renal artery length threshold are non-limiting examples and are not limited to the distance values discussed above. In addition, the main renal artery length anatomical complexity scores are non-limiting examples and are not limited to the main renal artery length anatomical complexity scores discussed above.

In some examples, the length of the main renal artery 15 may be compared to a distance from the main renal artery ostium 16 to a kidney hilum to determine the main renal artery length anatomical complexity score. For example, if the length of the main renal artery 15 is greater than or equal to an upper threshold percentage, such as 50%, of the distance from the main renal artery ostium 16 to the kidney hilum, processing circuitry 46 may determine the main renal artery length anatomical complexity score is given its lowest value, such as "1". If the length of the main renal artery 15 is less than or equal to a lower threshold percentage, such as 30%, of the distance from the main renal artery ostium 16 to the kidney hilum, processing circuitry 46 may determine the main renal artery length anatomical complexity score is given its highest value, such as "3". If the length of the main renal artery 15 is determined to be greater than the lower threshold percentage and less than the upper threshold percentage, processing circuitry 46 may determine the main renal artery length anatomical complexity score is given a value between its highest value and lowest value, such as "2". The upper threshold percentage and lower threshold percentage are non-limiting examples and are not limited to the percentage values discussed above. In addition, the main renal artery length anatomical complexity scores are non-limiting examples and are not limited to the main renal artery length anatomical complexity scores discussed above.

Processing circuitry 46 may generate a lower value for the main renal artery count anatomical complexity score based, at least in part, on a lower number of main renal arteries present for a respective kidney. For example, if processing circuitry 46 determines there are a number of main renal arteries less than or equal to a lower main renal artery count threshold, such as one, processing circuitry 46 may determine the main renal artery count anatomical complexity score is given the lowest value, such as "1". For example, if processing circuitry 46 determines, based on the renal arterial anatomy information, there is a single main renal artery, processing circuitry 46 determines the main renal artery count anatomical complexity score is given the lowest value. If processing circuitry 46 determines there are a number of main renal arteries greater than or equal to an upper main renal artery count threshold, such as four, processing circuitry 46 may determine to give the main renal artery count anatomical complexity score the highest value, such as "3". If processing circuitry 46 determines there are a number of renal arteries less than an upper main renal artery count threshold and greater than the lower main renal artery count threshold, processing circuitry 46 may determine to give the main renal artery count anatomical complexity score a value between its highest value and lowest value, such as "2". The lower main renal artery count threshold and upper main renal artery count threshold are non-limiting examples and are not limited to the count values discussed above. In addition, the main renal artery count anatomical complexity scores are non-limiting examples and are not limited to the main renal artery count anatomical complexity scores discussed above.

In some examples, processing circuitry 46 may determine a renal anatomical complexity score based on one or more of the main renal artery length anatomical complexity score or the main renal artery count anatomical complexity score. In some examples, processing circuitry 46 may determine a renal anatomical complexity score based on both the main renal artery length anatomical complexity score and the main renal artery count anatomical complexity score. In some examples, a renal anatomical complexity score for each respective kidney may be combined to determine a combined renal anatomical complexity score. For example, if processing circuitry 46 determines the main renal artery length anatomical complexity score is given its lowest value, such as "1", and determines the main renal artery count anatomical complexity score is given the lowest value, such as "1", processing circuitry 46 may determine the renal anatomical complexity score to be "2" or "1". For example, if processing circuitry 46 determines the main renal artery length anatomical complexity score is given its highest value, such as "3", and determines the main renal artery count anatomical complexity score is given the highest value, such as "3", processing circuitry 46 may determine the renal anatomical complexity score to be "6" or "9". In some examples, processing circuitry 46 may combine and/or weigh the main renal artery length anatomical complexity score and the main renal artery count anatomical complexity score in a variety of different ways to determine renal anatomical complexity score, such as adding the scores together, multiplying the scores together, or other variations. In addition, the renal anatomical complexity scores are non-limiting examples and are not limited to the renal anatomical complexity scores discussed above.

Processing circuitry 46 may determine a type of RDN therapy to apply to patient 18 based on the determined renal anatomical complexity score. A determined type of RDN therapy may include one or more features of therapy to be applied, such as complexity, duration, and/or modality (e.g., radiofrequency ablation, ultrasound ablation, cryoablation, chemical, etc.) of RDN therapy to be applied to the patient. Processing circuitry 46 may compare the determined renal anatomical complexity score to treatment thresholds to determine a type of RDN therapy to apply to patient 18. Computing device 18 may compare the determined renal anatomical complexity score to one or more of a lower treatment threshold or an upper treatment threshold. In some examples, a lower treatment threshold may be "2" and an upper treatment threshold may be "6". However, the lower treatment threshold and upper treatment threshold values are non-limiting examples and are not limited to the lower treatment threshold and upper treatment threshold values discussed throughout, as the lower treatment threshold and upper treatment threshold values may be a variety of other different values.

In response to the renal anatomical complexity score being less than or equal to a lower treatment threshold, processing circuitry 46 may determine to apply simplified RDN therapy, such as applying one or more ablations only to a trunk of the main renal artery. In response to the renal anatomical complexity score being greater than or equal to an upper treatment threshold, processing circuitry 46 may determine to apply a complex RDN therapy, such as applying one or more ablations to one or more primary and secondary branches of the main renal artery, in addition to applying one or more ablations to the trunk of the main renal artery. In response to the determined renal anatomical complexity score being between a lower treatment threshold and an upper treatment threshold, processing circuitry 46 may determine to apply an intermediate complexity RDN therapy, such as applying one or more ablations to the trunk of the main renal artery and one or more primary branches of the renal artery.

For example, if processing circuitry 46 determines the main renal artery length anatomical complexity score is given its lowest value, such as "1", and determines the main renal artery count anatomical complexity score is given the lowest value, such as "1", processing circuitry 46 may determine the renal anatomical complexity score to be "2 and then determine the type of RDN therapy to apply to the patient is an RDN therapy of applying one or more ablations to the trunk of the main renal artery only. In some examples, processing circuitry 46 may compare the renal anatomical complexity score to a lower treatment threshold. Processing circuitry 46 may determine to apply one or more ablations only to a trunk of the main renal artery in response to the renal anatomical complexity score being less than or equal to a lower treatment threshold. In this example, since processing circuitry 46 determined patient had a particular renal arterial anatomy that may benefit from an RDN treatment of applying one or more ablations to the trunk of the main renal artery only, this may reduce discomfort, inconvenience, and/or side effects in the patient while also reducing costs of RDN therapy.

As another example, if processing circuitry 46 determines the main renal artery length anatomical complexity score is given its highest value, such as "3", and determines the main renal artery count anatomical complexity score is given the highest value, such as "3", computing device may determine the renal anatomical complexity score to be "6". Processing circuitry 46 may determine that the RDN therapy to be applied to patient 18 may include one or more ablations to the trunk of the main renal artery and one or more ablations in the primary and secondary branches and/or accessory arteries of the main renal artery based on patient 18 having a renal anatomical complexity score greater than or equal to an upper treatment threshold, such as 6.

As another example, if processing circuitry 46 determines the main renal artery length anatomical complexity score is given its highest value, such as "3", and determines the main renal artery count anatomical complexity score is given the lowest value, such as "1", computing device may determine the renal anatomical complexity score to be "4". In response to determining the renal anatomical complexity score to be "4", processing circuitry 46 may determine to apply one or more ablations to the trunk of the main renal artery and a primary branch of the main renal artery.

In some examples, processing circuitry 46 may determine a respective renal anatomical complexity score based on renal arterial anatomy information associated with a respective left or right kidney independently. In some examples, processing circuitry 46 may determine a renal anatomical complexity score based on renal arterial anatomy information associated with left and right kidneys. For example, processing circuitry 46 may determine a renal anatomical complexity score based, at least in part, on a ratio of main renal artery length associated with the right kidney vs main renal artery length associated with the left kidney.

Processing circuitry 46 may output an indication of the determined type of RDN therapy to apply to patient 18. For example, processing circuitry 46 may output an indication of the type of RDN therapy to apply to patient 18 to be displayed on user interface 44 of the processing circuitry 46 to indicate to a clinician the type of RDN therapy to apply to patient 18. In some examples, processing circuitry 46 may output an indication of the type of RDN therapy to apply to patient 18 to another computing device to indicate to a clinician the type of RDN therapy to apply to patient 18. For example, an output indication may include one or more images of patient 18 anatomy with preferred ablation points and/or regions identified or overlaid on the one or more images. Accordingly, a clinician may be able to apply the of RDN therapy that is necessary for an individual patient, which may result in reduction of unnecessary ablations being applied in RDN procedures. This may result in quicker procedures, which may require less anesthetic and/or less energy, as well as reducing patient discomfort.

Determining a type of RDN therapy to be performed on a particular patient may result in less wasted health care resources, less patient inconvenience and discomfort, less potential unnecessary side effects, etc. This may help reduce unnecessary health care costs and reduce invasive procedures on patients that do not require complex RDN therapy to be applied.

The above-described example techniques assign a numerical score based on number of arteries or length of arteries. However, the example techniques are not so limited. There may be other ways in which to determine a renal anatomical complexity score, including machine learning based techniques.

For instance, processing circuitry 46 may be configured to execute an AI engine that operates according to one or more models, such as machine learning models. Machine learning models may include any number of different types of machine learning models, such as neural networks, deep neural networks, dense neural networks, and the like. Although described with respect to machine learning models, the techniques described in this disclosure are also applicable to other types of AI models, including rule-based models, finite state machines, and the like.

Machine learning may generally enable a computing device to analyze input data and identify an action to be performed responsive to the input data. Each machine learning model may be trained using training data that reflects likely input data. The training data may be labeled or unlabeled (meaning that the correct action to be taken based on a sample of training data is explicitly stated or not explicitly stated, respectively).

The training of the machine learning model may be guided (in that a designer, such as a computer programmer, may direct the training to guide the machine learning model to identify the correct action in view of the input data) or unguided (in that the machine learning model is not guided by a designer to identify the correct action in view of the input data). In some instances, the machine learning model is trained through a combination of labeled and unlabeled training data, a combination of guided and unguided training, or possibly combinations thereof. Examples of machine learning include nearest neighbor, naive Bayes, decision trees, linear regression, support vector machines, neural networks, k-Means clustering, Q-learning, temporal difference, deep adversarial networks, evolutionary algorithms or other supervised, unsupervised, semi-supervised, or reinforcement learning algorithms to train one or more models.

Computing device 12 may utilize machine learning, such as a deep learning algorithm or model (e.g., a neural network or deep belief network), to generate (e.g., determine) a result indicative of a type of RDN therapy that should be applied to patient 18. The generated (e.g., determined) result may be output as an indication of a type of RDN therapy to be applied to the patient.

A ML training device other than computing device 12 may be configured to train the deep learning model to generate a trained model that is then executed on computing device 12. That is, memory 40 may store the trained model, and processing circuitry 46 may execute the trained model.

The ML training device may train a deep learning model to represent a relationship of renal arterial anatomy information and other patient metrics of patients to a type of RDN therapy to be applied. The ML training device may train a deep learning model to represent a relationship of renal anatomical complexity score and other patient metrics of patients to a type of RDN therapy to be applied. For example, the ML training device may train the deep learning model using imaging data of renal arterial anatomy, renal arterial anatomy information and/or renal anatomical complexity score, patient metrics, and responsive of a type of renal denervation therapy from other patients. In some examples, the ML training device may train the deep leaning model by adjusting the weights of a hidden layer of a neural network model to balance the contribution of each input (e.g., characteristics of the imaging data, renal arterial anatomy information and/or renal anatomical complexity score) according to how responsive a patient was to a type of renal denervation therapy.

In some examples, a training set may include renal arterial anatomy information, information of the type RDN therapy applied, and/or other patient information. In some examples for the training set, a reduction in blood pressure for a patient greater than a first threshold may be considered a good result, a reduction in blood pressure for a patient less than a first threshold but greater than a second threshold may be considered an average result, and a reduction of blood pressure for a patient less than a second threshold may be considered a bad result.

From a training set, a machine learning model may receive patient renal arterial anatomy information and/or other patent information and determine a type RDN therapy that provides optimal results.

Once the deep learning model is trained, computing device 12 may obtain and apply the trained model. For instance, memory 40 may store the trained model for execution by processing circuitry 46 and application on the collected imaging data, renal arterial anatomy information and/or renal anatomical complexity score and/or a plurality of values representative of respective patient metrics for the patient, to the trained deep learning model. Example patient metrics may include an age of the patient, a gender of the patient, an ethnic background of the patient, a weight of the patient, a height of the patient, a diet of the patient, an activity level of the patient, or a stress level of the patient. The output of the deep learning model may include the result that indicates a type of RDN therapy to apply to patient. For example, the result may be a probability that the patient would achieve a target reduction in hypertension in response to different types of RDN therapy. In some examples, the result may be indicative of the magnitude of the reduction in systemic blood pressure that the patient may realize after RDN therapy. Computing device 12 may display the result to a clinician to aid in determining a type of renal denervation therapy to apply to the patient.

In some examples, computing device 12 may send the collected imaging data, received renal arterial anatomy information and/or renal anatomical complexity score, to a separate computing system to utilize the separate computing system to perform the machine learning, such as a deep learning algorithm or model, on the received renal arterial anatomy information and/or renal anatomical complexity score.

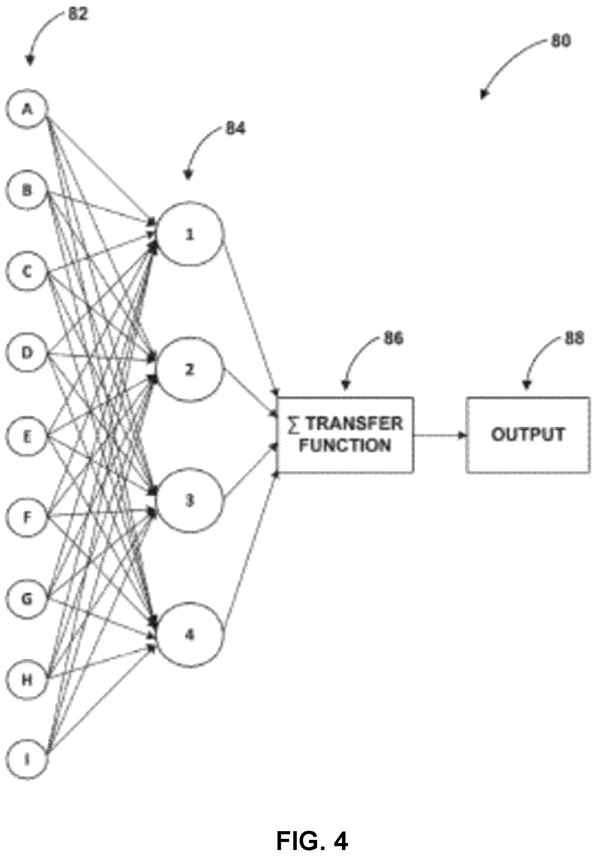

FIG. 4 is a conceptual diagram illustrating an example neural network 80 configured to determine a type of renal denervation therapy to apply to a patient diagnosed with hypertension. Neural network 80 is an example of a deep learning model, or deep learning algorithm, trained to generate a result indicative of renal denervation therapy efficacy. As discussed above, other types of machine learning and deep learning models or algorithms may be utilized in other examples. Computing device 12 may train, store, and/or utilize neural network 80, but other devices may apply inputs associated with a particular patient to neural network 80 in other examples. Neural network 80 may be stored by computing device 12.

As shown in the example of FIG. 4, neural network 80 comprises three layers. These three layers include input layer 82, hidden layer 84, and output layer 86. Output layer 88 comprises the output from the transfer function of output layer 86. Input layer 82 represents each of the input values A through I provided to neural network 80. The input values may be values for patient metrics such as an age of the patient, a gender of the patient, an ethnic background of the patient, a weight of the patient, a height of the patient, a diet of the patient, an activity level of the patient, and/or a stress level of the patient. The input values may be numerical or categorical as appropriate for each patient metric. In some examples, values for all of these patient metrics may be incorporated into neural network 80.

In addition, some input values of input layer 82 may include one or more characteristics of imaging data, renal arterial anatomy information and/or renal anatomical complexity score, from the computing device 12. For example, the main renal artery length anatomical complexity score and/or the main renal artery count anatomical complexity score. These characteristics may be input into input layer 82.

Each of the input values for each node in the input layer 82 is provided to each node of hidden layer 84. In the example of FIG. 4, hidden layer 84 include four nodes, but fewer or greater number of nodes may be used in other examples. Each input from input layer 82 is multiplied by a weight and then summed at each node of hidden layer 84. During training of neural network 80, the weights for each input are adjusted to establish the relationship between the renal arterial anatomy information and/or renal anatomical complexity score based to efficacy of a type of renal denervation therapy. In some examples, two or more hidden layers may be incorporated into neural network 80, where each layer includes the same or different number of nodes.

The result of each node within hidden layer 84 is applied to the transfer function of output layer 86. The transfer function may be linear or non-linear, depending on the number of layers within neural network 80. Example non-linear transfer functions may be a sigmoid function or a rectifier function. The output 88 of the transfer function may be the result that is generated by computing device 12 in response to applying renal arterial anatomy information and/or renal anatomical complexity score for the patient to neural network 80. A deep learning model, such as neural network 80, may enable a computing system such as computing device 12 to determine a type of renal denervation therapy to apply to a patient using a variety of values representing the condition of a particular patient.

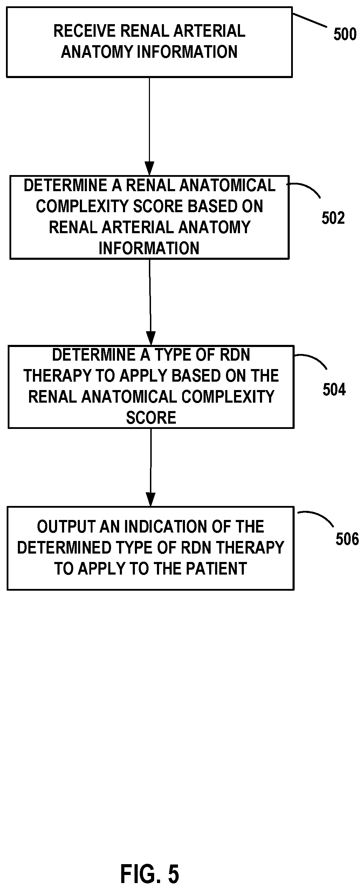

FIG. 5 is a flow diagram illustrating an example technique for operating system 10. As indicated by FIG. 5, processing circuitry 46 may receive renal arterial anatomy information (500). The renal arterial anatomy information may include one or more of a length of a main renal artery or a number of main renal arteries. Processing circuitry 46 may determine a renal anatomical complexity score based on renal arterial anatomy information (502). In some examples, processing circuitry 46 may determine a main renal artery length anatomical complexity score based on a length of the main renal artery and/or determine a main renal artery count anatomical complexity score by comparing the number of main renal arteries to a main renal artery count threshold. Processing circuitry 46 may determine the renal anatomical complexity score based on the main renal artery length anatomical complexity score and the main renal artery count anatomical complexity score.

Processing circuitry 46 may determine a type of RDN therapy to apply based on the renal anatomical complexity score (504). For example, processing circuitry 46 may determine to apply one or more ablations only to the trunk main renal artery in response to the renal anatomical complexity score being less than or equal to a lower treatment threshold. Processing circuitry 46 may determine to apply one or more ablations to the trunk of the main renal artery and to primary and secondary branches of the main renal artery in response to the renal anatomical complexity score being greater than or equal to an upper treatment threshold. Processing circuitry 46 may determine to apply one or more ablations to the trunk of the main renal artery and to a primary branch in response to the renal anatomical complexity score being between a lower treatment threshold and an upper treatment threshold. Processing circuitry 46 may output an indication of the determined type of RDN therapy to apply to patient 18 (506).

The techniques described in this disclosure may be implemented, at least in part, in hardware, software, firmware or any combination thereof. For example, various aspects of the described techniques may be implemented within one or more processors or processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also perform one or more of the techniques of this disclosure.

Such hardware, software, and firmware may be implemented within the same device or within separate devices to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

## Claims

1. A system (10) comprising:
a memory (40); and
processing circuitry (46) coupled to the memory (40), the processing circuitry (46) configured to:
receive renal arterial anatomy information of a patient (18);
determine a renal anatomical complexity score based on the renal arterial anatomy information;
determine a type of renal denervation therapy to apply to the patient (18) based on the renal anatomical complexity score; and
output an indication of the determined type of renal denervation therapy to apply to the patient (18).

2. The system (10) of claim 1, wherein the renal arterial anatomy information includes one or more of a length of a main renal artery or a number of main renal arteries.

3. The system (10) of claim 2, wherein the processing circuitry (46) is further configured to:
determine a main renal artery length anatomical complexity score based on the length of the main renal artery.

4. The system (10) of claim 3, wherein the processing circuitry (46) is further configured to:
determine the main renal artery length anatomical complexity score by comparing the length of the main renal artery to a main renal artery length threshold, wherein the length of the main renal artery is a length from a main renal artery ostium of the main renal artery to a primary bifurcation point of the main renal artery.

5. The system (10) of claim 4, wherein the main renal artery length anatomical complexity score is inversely related to the length of the main renal artery from the main renal artery ostium to the primary bifurcation point.

6. The system (10) of any of claims 2-5, wherein the processing circuitry (46) is further configured to:
determine a main renal artery count anatomical complexity score by comparing the number of main renal arteries to a main renal artery count threshold.

7. The system (10) of claim 6, wherein the processing circuitry (46) is further configured to:
determine the renal anatomical complexity score based on the main renal artery count anatomical complexity score.

8. The system (10) of claim 6, wherein the processing circuitry (46) is further configured to:
determine the renal anatomical complexity score based on the main renal artery length anatomical complexity score and the main renal artery count anatomical complexity score.

9. The system (10) of any of claims 2-8, wherein to determine the type of renal denervation therapy to apply, the processing circuitry (46) is further configured to:
determine to apply one or more ablations only to a trunk of the main renal artery in response to the renal anatomical complexity score being less than or equal to a lower treatment threshold.

10. The system (10) of any of claims 2-9, wherein to determine the type of renal denervation therapy to apply, the processing circuitry (46) is further configured to:
determine to apply one or more ablations to a trunk of the main renal artery and primary and secondary branches of the main renal artery in response to the renal anatomical complexity score being greater than or equal to an upper treatment threshold.

11. The system (10) of any of claims 2-10, wherein to determine the type of renal denervation therapy to apply, the processing circuitry (46) is further configured to:
determine to apply one or more ablations to a trunk of the main renal artery and to a primary branch of the main renal artery in response to the renal anatomical complexity score being between a lower treatment threshold and an upper treatment threshold.

12. The system (10) of any of claims 1-11, wherein a computing device (12) comprises the memory (40) and the processing circuitry (46), the system (10) further comprising:
an imaging device (30) configured to collect data indicative of the renal arterial anatomy information of a patient (18), and
wherein the computing device processing circuitry (46) is configured to receive the renal arterial anatomy information from the imaging device.

13. A non-transitory computer-readable storage medium comprising instructions that, when executed by processing circuitry (46), cause the processing circuitry (46) to:
receive renal arterial anatomy information of a patient (18);
determine a renal anatomical complexity score based on the renal arterial anatomy information;
determine a type of renal denervation therapy to apply to the patient (18) based on the renal anatomical complexity score; and
output an indication of the determined type of renal denervation therapy to apply to the patient (18).

14. The non-transitory computer-readable storage medium of claim 13, wherein the renal arterial anatomy information includes one or more of a length of a main renal artery or a number of main renal arteries.

15. The non-transitory computer-readable storage medium of claim 14, that, when executed by processing circuitry (46), further causes the processing circuitry (46) to:
determine a main renal artery length anatomical complexity score by comparing the length of the main renal artery to a main renal artery length threshold, wherein the length of the main renal artery is a length from a main renal artery ostium of the main renal artery to a primary bifurcation point of the main renal artery.

## Patentansprüche

1. System (10), umfassend:
einen Speicher (40); und
eine Verarbeitungsschaltung (46), die mit dem Speicher (40) gekoppelt ist, wobei die Verarbeitungsschaltung (46) ausgelegt ist zum:
Empfangen von Informationen über die Anatomie der Nierenarterien eines Patienten (18);
Bestimmen eines renalen anatomischen Komplexitäts-Scores basierend auf den Informationen über die Anatomie der Nierenarterien;
Bestimmen einer Art von renaler Denervationstherapie zur Anwendung an dem Patienten (18) basierend auf dem renalen anatomischen Komplexitäts-Score; und
Ausgeben eines Hinweises auf die bestimmte Art der renalen Denervationstherapie zur Anwendung an dem Patienten (18).

2. System (10) nach Anspruch 1, wobei die Informationen über die Anatomie der Nierenarterien eine oder mehrere einer Länge einer Hauptnierenarterie oder einer Anzahl von Hauptnierenarterien umfassen.

3. System (10) nach Anspruch 2, wobei die Verarbeitungsschaltung (46) ferner ausgelegt ist zum: Bestimmen eines anatomischen Komplexitäts-Scores für die Länge einer Hauptnierenarterie basierend auf der Länge der Hauptnierenarterie.

4. System (10) nach Anspruch 3, wobei die Verarbeitungsschaltung (46) ferner ausgelegt ist zum: Bestimmen des anatomischen Komplexitäts-Scores für die Länge der Hauptnierenarterie durch Vergleichen der Länge der Hauptnierenarterie mit einem Längenschwellenwert für die Hauptnierenarterie, wobei die Länge der Hauptnierenarterie eine Länge von einem Hauptnierenarterienostium der Hauptnierenarterie bis zu einem primären Bifurkationspunkt der Hauptnierenarterie ist.

5. System (10) nach Anspruch 4, wobei der anatomische Komplexitäts-Score für die Länge der Hauptnierenarterie umgekehrt proportional zu der Länge der Hauptnierenarterie vom Ostium der Hauptnierenarterie bis zum primären Bifurkationspunkt ist.

6. System (10) nach einem der Ansprüche 2-5, wobei die Verarbeitungsschaltung (46) ferner ausgelegt ist zum: Bestimmen eines anatomischen Komplexitäts-Scores der Anzahl der Hauptnierenarterien durch Vergleichen der Anzahl der Hauptnierenarterien mit einem Schwellenwert für die Anzahl der Hauptnierenarterien.

7. System (10) nach Anspruch 6, wobei die Verarbeitungsschaltung (46) ferner ausgelegt ist zum: Bestimmen des renalen anatomischen Komplexitäts-Scores basierend auf dem anatomischen Komplexitäts-Score der Anzahl der Hauptnierenarterien.

8. System (10) nach Anspruch 6, wobei die Verarbeitungsschaltung (46) ferner ausgelegt ist zum: Bestimmen des renalen anatomischen Komplexitäts-Scores basierend auf dem anatomischen Komplexitäts-Score der Länge der Hauptnierenarterie und dem anatomischen Komplexitäts-Score der Anzahl der Hauptnierenarterien.

9. System (10) nach einem der Ansprüche 2-8, wobei zur Bestimmung der Art der anzuwendenden renalen Denervationstherapie die Verarbeitungsschaltung (46) ferner ausgelegt ist zum:
Bestimmen, dass eine oder mehrere Ablationen nur an einem Rumpf der Hauptnierenarterie angewendet werden sollen, als Reaktion darauf, dass der renale anatomische Komplexitäts-Score kleiner als ein unterer Behandlungsschwellenwert ist oder diesem gleicht.

10. System (10) nach einem der Ansprüche 2-9, wobei zur Bestimmung der Art der anzuwendenden renalen Denervationstherapie die Verarbeitungsschaltung (46) ferner ausgelegt ist zum:
Bestimmen, dass eine oder mehrere Ablationen an einem Rumpf der Hauptnierenarterie und primären und sekundären Ästen der Hauptnierenarterie angewendet werden sollen, als Reaktion darauf, dass der renale anatomische Komplexitäts-Score größer als ein oberer Behandlungsschwellenwert ist oder diesem gleicht.

11. System (10) nach einem der Ansprüche 2-10, wobei zur Bestimmung der Art der anzuwendenden renalen Denervationstherapie die Verarbeitungsschaltung (46) ferner ausgelegt ist zum:
Bestimmen, dass eine oder mehrere Ablationen an einem Rumpf der Hauptnierenarterie und an einem primären Ast der Hauptnierenarterie angewendet werden sollen, als Reaktion darauf, dass der renale anatomische Komplexitäts-Score zwischen einem unteren Behandlungsschwellenwert und einem oberen Behandlungsschwellenwert liegt.

12. System (10) nach einem der Ansprüche 1-11, wobei eine Computervorrichtung (12) den Speicher (40) und die Verarbeitungsschaltung (46) umfasst, wobei das System (10) ferner umfasst:
eine Bildgebungsvorrichtung (30), die zum Sammeln von Daten ausgelegt ist, die auf die Informationen über die Anatomie der Nierenarterien eines Patienten hinweisen (18), und
wobei die Verarbeitungsschaltung (46) der Computervorrichtung zum Empfangen der Informationen über die Anatomie der Nierenarterien von der Bildgebungsvorrichtung ausgelegt ist.

13. Nicht-flüchtiges computerlesbares Speichermedium, umfassend Anweisungen, die bei Ausführung durch die Verarbeitungsschaltung (46) bewirken, dass die Verarbeitungsschaltung (46):
Empfangen von Informationen über die Anatomie der Nierenarterien eines Patienten (18);
Bestimmen eines renalen anatomischen Komplexitäts-Scores basierend auf den Informationen über die Anatomie der Nierenarterien;
Bestimmen einer Art von renaler Denervationstherapie zur Anwendung an dem Patienten (18) basierend auf dem renalen anatomischen Komplexitäts-Score; und
Ausgeben eines Hinweises auf die bestimmte Art der renalen Denervationstherapie zur Anwendung an dem Patienten (18).

14. Nicht-flüchtiges computerlesbares Speichermedium nach Anspruch 13, wobei die Informationen über die Anatomie der Nierenarterien eine oder mehrere einer Länge einer Hauptnierenarterie oder einer Anzahl der Hauptnierenarterien umfassen.

15. Nicht-flüchtiges computerlesbares Speichermedium nach Anspruch 14, das bei Ausführung durch die Verarbeitungsschaltung (46) ferner bewirkt, dass die Verarbeitungsschaltung (46):
einen anatomischen Komplexitäts-Score für die Länge der Hauptnierenarterie durch Vergleichen der Länge der Hauptnierenarterie mit einem Längenschwellenwert für die Hauptnierenarterie bestimmt, wobei die Länge der Hauptnierenarterie eine Länge von einem Hauptnierenarterienostium der Hauptnierenarterie bis zu einem primären Bifurkationspunkt der Hauptnierenarterie ist.

## Revendications

1. Système (10) comprenant :
une mémoire (40) ; et
un circuit de traitement (46) couplé à la mémoire (40), le circuit de traitement (46) étant configuré pour :
recevoir des informations sur l'anatomie artérielle rénale d'un patient (18) ;
déterminer un score de complexité anatomique rénale sur la base des informations sur l'anatomie artérielle rénale ;
déterminer un type de thérapie de dénervation rénale à appliquer au patient (18) sur la base du score de complexité anatomique rénale ; et
émettre une indication du type de thérapie de dénervation rénale déterminé à appliquer au patient (18).

2. Système (10) selon la revendication 1, les informations sur l'anatomie artérielle rénale comprenant une ou plusieurs d'une longueur d'une artère rénale principale ou d'un nombre d'artères rénales principales.

3. Système (10) selon la revendication 2, le circuit de traitement (46) étant en outre configuré pour :
déterminer un score de complexité anatomique de longueur d'artère rénale principale sur la base de la longueur de l'artère rénale principale.

4. Système (10) selon la revendication 3, le circuit de traitement (46) étant en outre configuré pour :
déterminer le score de complexité anatomique de longueur d'artère rénale principale en comparant la longueur de l'artère rénale principale à un seuil de longueur d'artère rénale principale, la longueur de l'artère rénale principale étant une longueur entre l'ostium d'artère rénale principale de l'artère rénale principale et un point de bifurcation primaire de l'artère rénale principale.

5. Système (10) selon la revendication 4, le score de complexité anatomique de longueur d'artère rénale principale étant inversement proportionnel à la longueur de l'artère rénale principale entre l'ostium d'artère rénale principale et le point de bifurcation primaire.

6. Système (10) selon l'une quelconque des revendications 2 à 5, le circuit de traitement (46) étant en outre configuré pour :
déterminer un score de complexité anatomique du nombre d'artères rénales principales en comparant le nombre d'artères rénales principales à un seuil de nombre d'artères rénales principales.

7. Système (10) selon la revendication 6, le circuit de traitement (46) étant en outre configuré pour :
déterminer le score de complexité anatomique rénale sur la base du score de complexité anatomique du nombre d'artères rénales principales.

8. Système (10) selon la revendication 6, le circuit de traitement (46) étant en outre configuré pour :
déterminer le score de complexité anatomique rénale sur la base du score de complexité anatomique de longueur d'artère rénale principale et du score de complexité anatomique du nombre d'artères rénales principales.

9. Système (10) selon l'une quelconque des revendications 2 à 8, pour déterminer le type de thérapie de dénervation rénale à appliquer, le circuit de traitement (46) étant en outre configuré pour :
déterminer d'appliquer une ou plusieurs ablations uniquement à un tronc de l'artère rénale principale en réponse au fait que le score de complexité anatomique rénale est inférieur ou égal à un seuil de traitement inférieur.

10. Système (10) selon l'une quelconque des revendications 2 à 9, pour déterminer le type de thérapie de dénervation rénale à appliquer, le circuit de traitement (46) étant en outre configuré pour :
déterminer d'appliquer une ou plusieurs ablations à un tronc de l'artère rénale principale et aux branches primaire et secondaire de l'artère rénale principale en réponse au fait que le score de complexité anatomique rénale est supérieur ou égal à un seuil de traitement supérieur.

11. Système (10) selon l'une quelconque des revendications 2 à 10, pour déterminer le type de thérapie de dénervation rénale à appliquer, le circuit de traitement (46) étant en outre configuré pour :
déterminer d'appliquer une ou plusieurs ablations à un tronc de l'artère rénale principale et à une branche primaire de l'artère rénale principale en réponse au fait que le score de complexité anatomique rénale est compris entre un seuil de traitement inférieur et un seuil de traitement supérieur.

12. Système (10) selon l'une quelconque des revendications 1 à 11, un dispositif informatique (12) comprenant la mémoire (40) et le circuit de traitement (46), le système (10) comprenant en outre :
un dispositif d'imagerie (30) configuré pour collecter des données indicatives des informations sur l'anatomie artérielle rénale d'un patient (18), et
le circuit de traitement du dispositif informatique (46) étant configuré pour recevoir les informations sur l'anatomie artérielle rénale provenant du dispositif d'imagerie.

13. Support de stockage non transitoire lisible par ordinateur comprenant des instructions qui, lorsqu'elles sont exécutées par un circuit de traitement (46), amènent le circuit de traitement (46) à :
recevoir des informations sur l'anatomie artérielle rénale d'un patient (18) ;
déterminer un score de complexité anatomique rénale sur la base des informations sur l'anatomie artérielle rénale ;
déterminer un type de thérapie de dénervation rénale à appliquer au patient (18) sur la base du score de complexité anatomique rénale ; et
émettre une indication du type de thérapie de dénervation rénale déterminé à appliquer au patient (18).

14. Support de stockage non transitoire lisible par ordinateur selon la revendication 13, les informations sur l'anatomie artérielle rénale comprenant une ou plusieurs d'une longueur d'une artère rénale principale ou d'un nombre d'artères rénales principales.

15. Support de stockage non transitoire lisible par ordinateur selon la revendication 14, qui, lorsqu'il est exécuté par un circuit de traitement (46), amène en outre le circuit de traitement (46) à :
déterminer un score de complexité anatomique de longueur d'artère rénale principale en comparant la longueur de l'artère rénale principale à un seuil de longueur d'artère rénale principale, la longueur de l'artère rénale principale étant une longueur entre l'ostium d'artère rénale principale de l'artère rénale principale et un point de bifurcation primaire de l'artère rénale principale.
